(19) Europäisches Patentamt
European Patent Office
Office européen des brevets

(11) Veröffentlichungsnummer: **0 273 270 B1**

(12) **EUROPÄISCHE PATENTSCHRIFT**

(45) Veröffentlichungstag der Patentschrift: **08.04.92**

(51) Int. Cl.5: **C07D 495/04**, C07D 491/048, //(C07D495/04,333:00,235:00), (C07D491/048,307:00,235:00)

(21) Anmeldenummer: **87118342.2**

(22) Anmeldetag: **10.12.87**

Die Akte enthält technische Angaben, die nach dem Eingang der Anmeldung eingereicht wurden und die nicht in dieser Patentschrift enthalten sind.

# (54) Verfahren zur Herstellung von (+)-Biotin.

(30) Priorität: **18.12.86 CH 5051/86**

(43) Veröffentlichungstag der Anmeldung: **06.07.88 Patentblatt 88/27**

(45) Bekanntmachung des Hinweises auf die Patenterteilung: **08.04.92 Patentblatt 92/15**

(84) Benannte Vertragsstaaten: **AT BE CH DE ES FR GB IT LI LU NL SE**

(56) Entgegenhaltungen:
**EP-A- 0 081 047**
**EP-A- 0 084 377**
**EP-A- 0 154 225**
**EP-A- 0 161 580**
**EP-A- 0 173 185**
**EP-A- 0 270 076**
**DE-B- 2 058 234**
**DE-B- 2 058 248**
**DE-B- 2 331 244**

(73) Patentinhaber: **LONZA AG**
**Gampel/Wallis(CH)**

(72) Erfinder: **McGarrity, John, Dr.**
**Rathausstrasse 5**
**Visp Kanton Wallis(CH)**
Erfinder: **Tenud, Leander, Dr.-Ing. Chem. ETH.**
**Tirlerstrasse 4**
**Visp Kanton Wallis(CH)**

(74) Vertreter: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**W-8000 München 40(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein neues Verfahren zur Herstellung von (+)-Biotin.

(+)-Biotin ist als menschliches Vitamin, als Vitamin H, bekannt. Es wird aber auch als pharmazeutische Wirksubstanz zur Behandlung von Dermatose und als Futtermittelzusatz mit wachstumssteigernder Wirkung für Nutztiere eingesetzt.

Zur Herstellung von (+)-Biotin wurden verschiedene Verfahren beschrieben.

Aus der US-PS 2 489 232 ist ein Verfahren bekannt, gemäss welchem racemisches Biotin hergestellt wird. Da jedoch bekanntlich nur das optisch aktive (+)-Biotin biologisch aktiv ist, musste das so hergestellte racemische Biotin anschliessend noch in die optischen Antipoden aufgetrennt werden. Einerseits werden hierbei alle Reaktionsstufen mit racemischem Material durchgeführt, wodurch die doppelte Menge an Substanzen verarbeitet werden müssen. Andererseits ist die Spaltung von racemischem Biotin in die entsprechenden Antipoden ein recht kompliziertes Verfahren, welches zudem noch unrentabel ist, da der unerwünschte Antipode praktisch nicht mehr racemisiert und in den Prozess zurückgeführt werden kann.

Eine Verbesserung dieses Verfahrens ist aus der US-PS 2 489 235 bekannt. Hierbei wird nunmehr die Racematspaltung bereits auf einer früheren Stufe durchgeführt, wobei jedoch auch diesem Verfahren immer noch der Nachteil anhaftet, dass die meisten Reaktionsstufen mit racemischem Material durchgeführt werden und, dass auch hier der bei dieser Spaltung anfallende unerwünschte Antipode ebenfalls praktisch nicht mehr racemisiert und in den Prozess zurückgeführt werden kann.

M.Murakami und Mitarbeiter haben ein verbessertes Verfahren zur Herstellung von dl-Biotin entwickelt (vgl. JP-PS 31 669/1970, 37 775/1970, 37 776/1970 und 3 580/1971). Die Verbesserung besteht in der Einführung einer Carboxybutylgruppe in die 4-Stellung des dl-1,3-Dibenzylhexahydrothieno-[3,4-d]-imidazol-2,4-dions. Dieses Dion wird mit einem 1,4-Dihalogenmagnesiumbutan umgesetzt und anschliessend mit Kohlendioxid carboxyliert.

Eine weitere Verbesserung haben Gerecke et al, DE-PS 20 58 248, entwickelt, indem bereits auf einer früheren Stufe durch optische Spaltung eines Triethylaminsalzes der folgenden Formel, worin R einen Cholesterylrest bedeutet, oder eines Ephedrinsalzes der folgenden Formel, worin R einen Cyclohexylrest bedeutet

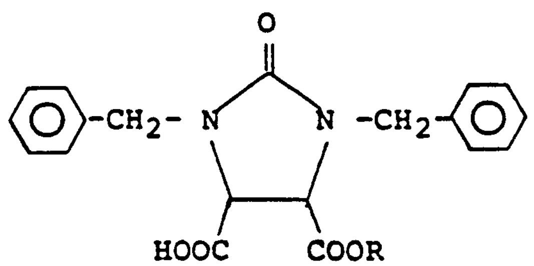

und durch weitere Ueberführung mit Alkalimetallborhydriden ein optisch aktives Lacton der Formel

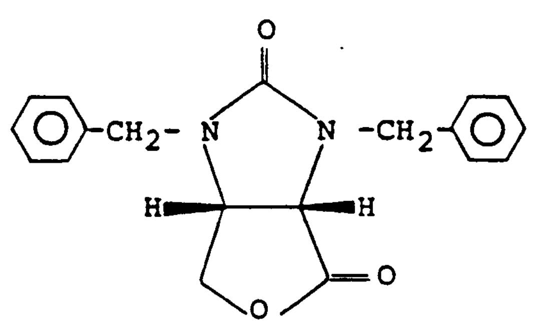

als optisch aktives Zwischenprodukt hergestellt wird.

Ein für eine technische Anwendung bedeutender Nachteil besteht in der Anwendung der teuren, optisch aktiven Verbindungen Cholesterin und Ephedrin sowie der teuren Alkalimetallborhydriden. Mit dem selben Nachteil, nämlich der Anwendung von teuren opt. aktiven Verbindungen, sind die Verfahren der EP-Anmeldungen 0 161 580 und 0 173 185 behaftet.

Ausserdem ist aus der EP-Anmeldung 0 154 225 bekannt, Biotin aus 1,3-Dibenzylhexahydro-1H-thienoimidazoldionen über eine spezielle Grignard-Reaktion mit einem Trioxaadamantylbutylmagnesiumbromid eine weiterführende Dehydratisierung und über die Abspaltung der entsprechenden Schutzgruppen herzustellen. Dieses Verfahren ist vor allem wegen seiner teuren Grignard-Verbindung für einen technischen Prozess ebenso nicht günstig.

Aufgabe der Erfindung war es, ein Verfahren zu finden, das es erlaubt, ausgehend von einem einfach verfügbaren Zwischenprodukt mit einem technisch durchführbaren Verfahren über möglichst wenige Reaktionsschritte (+)-Biotin herzustellen.

Diese Aufgabe wurde gelöst mit dem erfindungsgemässen Verfahren gemäss Patentanspruch 1 worin man Verbindungen der Formel

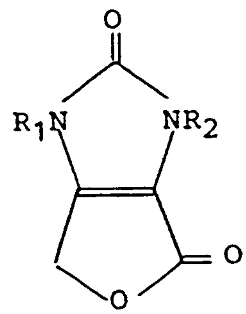

I

worin $R_1$ eine (R)- oder (S)-1-Phenylethylgruppe und $R_2$ Wasserstoff, eine Acetyl- oder Propionylgruppe, eine Benzoylgruppe, eine Benzylgruppe oder p-Methoxybenzylgruppe, eine Ethoxycarbonylgruppe, eine tert.-Butoxycarbonylgruppe, eine Benzyloxycarbonylgruppe, eine Phenoxycarbonylgruppe, eine Methoxymethylgruppe, eine Pyranylgruppe, eine Benzolsulfonylgruppe, eine p-Toluolsulfonylgruppe, eine Methylsulfonylgruppe, eine Diphenylphosphinylgruppe, eine Diethoxyphosphinylgruppe oder eine Trimethyl- oder tert.-Butyl-dimethylsilylgruppe bedeuten, katalytisch mit Wasserstoff hydriert, das gewünschte Diastereomer der Formel

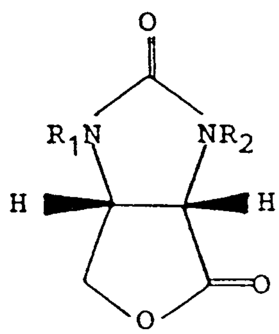

II

abtrennt, wenn $R_2$ = H die Schutzgruppe durch Umsetzung mit entsprechenden aliphatischen oder aromatischen Säurechloriden, aliphatischen oder aromatischen Carbonsäureanhydriden, Halogenameisensäurealkylestern, Benzylhalogeniden, 1-Alkoxyalkylhalogeniden, Enolethern, aromatischen oder aliphatischen Sulfonsäurehalogeniden, Diarylphosphinsäurehalogeniden, Phosphorsäuredialkylesterchloriden, Trialkylsilylhalogeniden oder Trialkylsilylacetamiden einführt, und weiter auf bekannte Weise durch Umsetzung mit einem Thiocarbonsäuresalzderivat in das entsprechende Thiolacton überführt, dieses entweder mit einer Grignard-Reaktion und anschließender Wasserabspaltung oder mit einer Verbindung der Formel

$(C_6H_5)_3P^{\oplus}(CH_2)_4COOR_3 . X^{\ominus}$ III

worin $R_3$ = H oder Alkyl mit 1 bis 4 C-Atomen und X ein Halogenatom bedeuten, in Gegenwart einer Base zu einer Verbindung der Formel

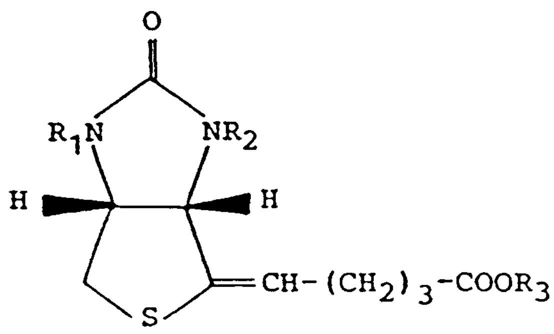

IV

worin $R_3$ oben genannte Bedeutung hat, umsetzt, in einem nächsten Schritt diese Verbindung katalytisch mit Wasserstoff hydriert und schließlich durch Abspalten der Schutzgruppen in das Endprodukt überführt.

Eine zentrale Bedeutung für das Verfahren kommt dabei den neuen 1H-Furo-[3,4-d]-imidazol-2,4-(3H,3aH)-dionen der Formel

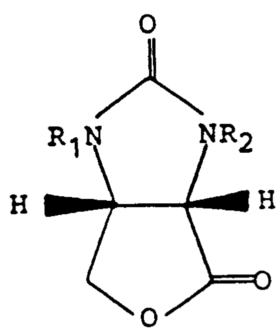

II

worin $R_1$ und $R_2$ die genannte Bedeutung haben, insbesondere aber dem (3aS,6aR)-[(R)-(1-Phenylethyl)]-3-benzyldihydro-1H-furo-[3,4-d]-imidazol-2,4(3H,3aH)-dion der Formel

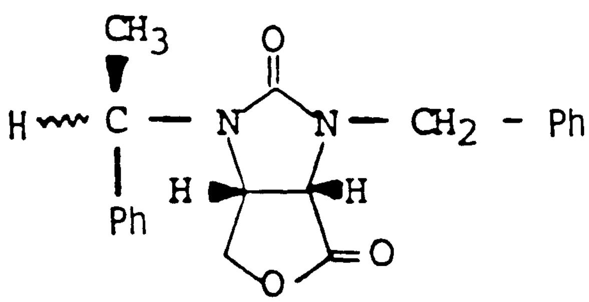

VII

zu.

Die Ausgangsprodukte der Formel

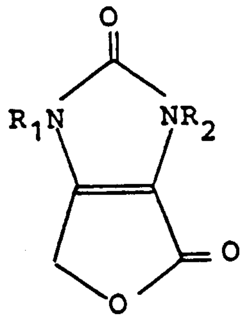

I

können gemäss CH-Patentgesuch Nr. 4790/86 aus Tetronsäure nach folgendem Schema hergestellt werden:

Schema 1

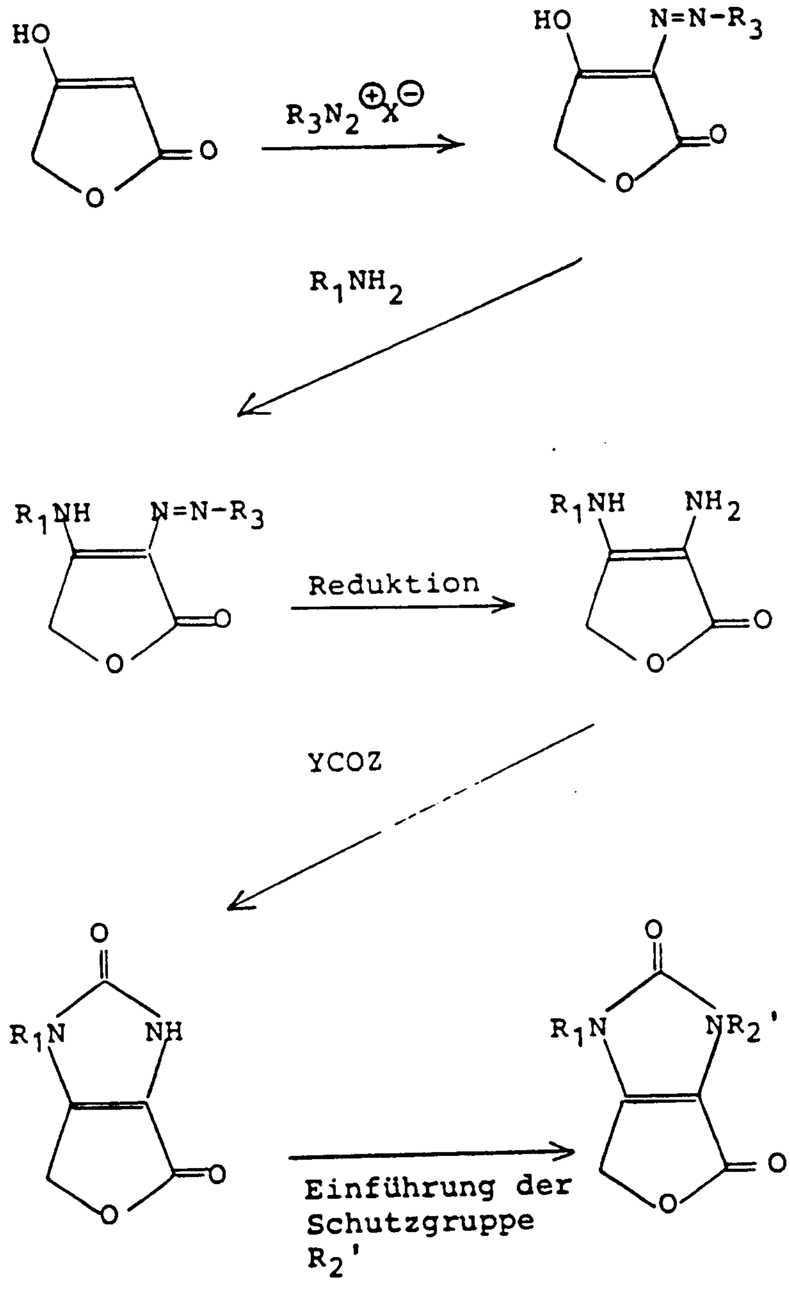


$R_3$ = Phenyl substituiert oder unsubstituiert
X = Halogen, $BF_4$, $HSO_4$
Y = Z = chlor, Imidazolyl
Y = Chlor
Z = Aryloxy oder Alkoxy
$R_1$, $R_2$ = die genannte Bedeutung
Bedeutet $R_2$ = H, kann entsprechend CH-Patentgesuch Nr. 4790/86 vor der erfindungsgemässen Hydrierung zuerst eine Schutzgruppe eingeführt werden.

Die Hydrierung von I wird katalytisch mit Wasserstoff durchgeführt, und zwar so, dass bevorzugt der Formel

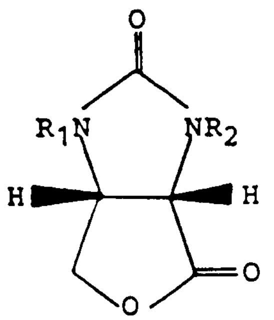

II

das eine (3aS, 6aR) Konfiguration besitzt, gebildet wird.

Zweckmässig werden Platin-, Palladium-, Ruthenium-, Rhodium-oder Nickelkatalysatoren, gegebenenfalls aufgebracht auf einen Träger wie Kohle, Siliciumdioxid, Aluminiumoxid, Aluminiumsilikat oder Calciumcarbonat, verwendet. Bevorzugter Vertreter aus dieser Gruppe ist Rhodium auf Aluminiumoxid als Träger. Ebenso einsetzbar sind homogene Katalysatoren, bestehend aus Rhodium oder Iridium mit Liganden wie Triphenylphosphin oder Cyclooctadien.

Die Stereoselektivität der Reaktion hängt stark von den Hydrierkatalysatoren und vom jeweiligen Substituent $R_1$ ab. So kann z.B. das Diastereomer II bei $R_1$ = (S)-1-Phenylethyl durch Einsatz eines Palladiumkatalysators und bei $R_1$ = (R)-1-Phenylethyl durch Einsatz eines Platinkatalysators erhalten werden.

Die Katalysatorkonzentration auf dem Träger liegt üblicherweise zwischen 1 und 100%, bevorzugt zwischen 1 und 10%. Zweckmässig wird der Katalysator in einer Menge von 1 bis 50 Mol%, bevorzugt zwischen 1 und 10 Mol%, der Reaktion zugesetzt.

Die Reaktion wird gewöhnlich in einem Lösungsmittel durchgeführt. Zweckmässig bieten sich dafür die niederen aliphatischen Alkohole wie z.B. Ethanol, Propanol, die niederen aliphatischen Carbonsäuren wie Essigsäure, Ether wie Diethylether, Tetrahydrofuran oder Dioxan, Carbonsäureamide wie Dimethylformamid und Dimethylacetamid, oder auch Carbonsäureester wie z.B. Essigsäureethylester oder auch halogenierte Kohlenwasserstoffe wie z.B. Dichlormethan, an.
Es kann unter Normaldruck gearbeitet werden, vorteilhaft ist aber die Reaktion unter Druck durchzuführen. Dieser kann sich in einem Bereich von zweckmässig 1 bis 70 bar, vorzugsweise 5 bis 30 bar, bewegen.

Selbstverständlich vom Lösungsmittel abhängig kann sich die Reaktionstemperatur zwischen -25 und +100 ° C bewegen.

Zum Erhalten des gewünschten optisch aktiven Diastereomers wird die Reaktionslösung zweckmässig zuerst vom Katalysator, dann vom Lösungsmittel befreit, der Rückstand anschliessend durch Umkristallisation in einem geeigneten Lösungsmittel vom unerwünschten Diastereomer (3aR-, 6aS-Isomer) getrennt.

Als geeignete Lösungsmittel werden zweckmässig Essigsäurealkylester wie Essigsäureethylester, niedere Alkohole wie Ethanol, oder auch Toluol eingesetzt.

Liegt II mit $R_2$ = H vor, wird vor der Umsetzung in das Thiolacton der Wasserstoff zweckmässig mit einer Schutzgruppe geschützt.

Die Einführung der Schutzgruppe $R_2'$ erfolgt zweckmässig durch Umsetzung von II mit entsprechenden aliphatischen oder aromatischen Säurechloriden, d.h. Acetylchlorid, Propionylchlorid oder Benzoylchlorid, mit Benzylhalogeniden oder mit p-Methoxybenzylchlorid, mit Halogenameisensäurealkylestern, wie Chlorameisensäureethylester, Chlorameisensäurebenzylester, Chlorameisensäuretert.-butylester oder Chlorameisensäurephenylester,mit Phosphorverbindungen wie Diphenylphosphinsäurechlorid oder Phosphorsäurediethylesterchlorid, mit aromatischen oder aliphatischen Sulfonsäurehalogeniden wie Methansulfonsäurechlorid, p-Toluolsulfonsäurechlorid oder Benzolsulfonsäurechlorid, mit Silylverbindungen wie Trialkylsilylacetamiden, Trimethylsilylchlorid, oder tert.-Butyl-dimethylsilylchlorid, mit 1-Alkoxyalkylhalogeniden wie Methoxymethylchlorid, oder mit Enolethern wie Dihydropyran. Ebenso geeignet sind die aliphatischen oder aromatischen Carbonsäureanhydride, wie Essigsäureanhydrid.

Das Einführen der Schutzgruppen kann nach bekannten Methoden erfolgen. Infolgedessen wird nicht näher darauf eingegangen.

Das mit Schutzgruppe $R_2'$ geschützte II kann dann durch Umsetzung mit einem Thiocarbonsäuresalzderivat in das entsprechende Thiolacton übergeführt werden.

Für $R_1$ und $R_2$ = Benzyl ist die Reaktion aus der DE-PS 20 58 234 bekannt.

Als Thiocarbonsäuresalzderivate werden zweckmässig die Erdalkali- oder Alkalisalze der aliphatischen oder aromatischen Thiocarbonsäuren, wie Kaliumthioacetat, Natriumthioacetat, Kaliumthiobenzoat oder

Natriumthiobenzoat, eingesetzt. Bevorzugt wird das Kaliumthioacetat eingesetzt.

Vorteilhaft werden zusätzlich Kronenether, wie z.B. der 18-Crown-6, als Katalysatoren eingesetzt.

Die Umsetzung wird vorteilhaft in einem inerten organischen Lösungsmittel bei Temperaturen zwischen 80 und 200 °C durchgeführt.

Als Lösungsmittel bieten sich, den Temperaturen entsprechend, hochsiedende Lösungsmittel, wie Dimethylformamid oder Dimethylacetamid als hochsiedende Amide, substituierte Aniline wie Lutidin, hochsiedende Amine, oder Toluol als hochsiedender Kohlenwasserstoff, an. Selbstverständlich können auch tiefersiedende Lösungsmittel eingesetzt werden. Zweckmässig wird die Reaktion dann unter Druck durchgeführt.

Nach für den Fachmann üblichem Aufarbeiten kann das gewünschte Thiolacton in guter Ausbeute erhalten werden.

Die Weiterumsetzung des Thiolactons mit einer Grignard-Reaktion und anschliessender Wasserabspaltung wurde bereits für $R_1$ und $R_2$ = Benzyl in der DE-PS 20 58 234 beschrieben.
Für das erfindungsgemässe Verfahren hat es sich als zweckmässig erwiesen, als Grignard-Reagens eine Verbindung der Formel

$XMg\text{-}(CH_2)_4\text{-}MgX$

worin X = Chlor oder Brom bedeutet, einzusetzen.
In einem zweiten Schritt kann dann durch Behandeln mit Kohlenoxid die Carboxylgruppe in die Seitenkette eingeführt werden. Schliesslich erfolgt die Ueberführung in die Verbindung IV durch Wasserabspaltung, zweckmässig in einem sauren Medium. Als vorteilhaft hat sich der Einsatz von p-Toluolsulfonsäure erwiesen.

Die Weiterumsetzung des Thiolactons kann auch mit III mittels einer Wittig-Reaktion zu IV erfolgen. Dies wurde für $R_1$ und $R_2$ = Benzyl in der EP-PS 0 084 377 bereits beschrieben.
So wird zweckmässig das Thiolacton durch Umsetzung mit III der Formel

$(C_6H_5)_3P^{\oplus}(CH_2)_4COOR_3X^{\ominus}$ III

worin $R_3$ = H oder Alkyl mit 1 bis 4 C-Atomen und X = Halogenatom bedeuten, in Gegenwart einer Base zu einer Verbindung der Formel

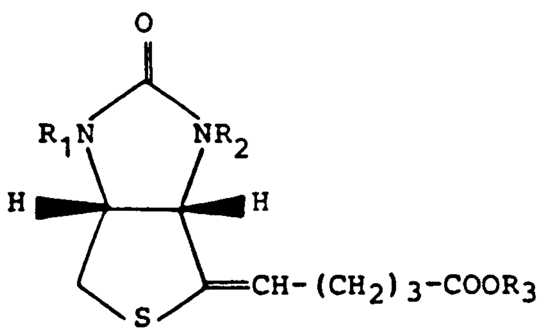

IV

umsetzt.
Bevorzugte Verbindung aus Formel III ist das Carboxybutyl-triphenylphosphoniumbromid.
Die Verbindungen werden, bezogen auf 1 Mol Thiolacton, zweckmässig in Mengen von 1 bis 5 Mol, vorzugsweise von 1,5 bis 2,5 Mol, eingesetzt.

Als zweckmässige Basen zur Bildung der Wittig-Reagens aus III finden Alkylalkalimetalle wie z.B. Butyllithium, Alkalimetallhydride wie z.B. Natriumhydrid oder Kaliumhydrid, Verbindungen der Formel

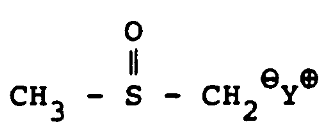

worin Y ein Alkalimetallatom bedeutet wie z.B. das Natriumdimethylsulfinylcarbanion, Alkalimetallalkoxide wie z.B. Natriummethoxid oder Kalium-tert-butoxid, Alkalimetallalkylamide wie z.B. Lithiumdiisopropylamid, Alkalimetallamide wie z.B. Natriumamid, Alkalimetallhydroxide wie z.B. Natriumhydroxid, Carbonate wie z.B. Natriumcarbonat, oder Siliciumverbindungen wie Alkalimetall-hexamethyldisilazide, Anwendung.

Bei der Wahl des Lösungsmittels ist darauf zu achten, dass dieses nicht mit den anderen Reaktionsteilnehmern reagiert. Geeignet sind dafür Ether wie Tetrahydrofuran, Dioxan, Dimethoxyethan oder Diethylether, aromatische Kohlenwasserstoffe wie Toluol, Benzol, chlorierte Kohlenwasserstoffe wie Dichlormethan, oder auch Dimethylsulfoxid.

Die Reaktionstemperatur sollte zweckmässig zwischen -50 bis +100 ° C, vorzugsweise zwischen 0 bis +80 ° C, liegen.

Nach beendeter Reaktion wird vorzugsweise angesäuert und auf übliche Weise aufgearbeitet. Die darauf folgende Reduktion von IV wird zweckmässig entsprechend EP 0 084 377 katalytisch mit Wasserstoff durchgeführt.

Als Katalysatoren dienen zweckmässig Palladium, Platin, Ruthenium, Rhodium, auf üblichen Trägermaterialien wie Kohle, Tonerde etc. Ebenso geeignet ist Raney-Nickel.

Die Katalysatormenge bewegt sich zweckmässig zwischen 1 und 20 Mol%, bezogen auf 1 Mol IV.

Vorteilhaft wird in aromatischen Kohlenwasserstoffen wie Toluol, aliphatischen Alkoholen wie Methanol, Ethanol, n-Propanol, Isopropanol, Carbonsäureestern wie Essigester, oder Ethern wie Tetrahydrofuran, Dioxan, oder auch in Wasser oder Essigsäure gearbeitet.

Den Wasserstoffdruck wählt man zweckmässig in einem Bereich von 5 bis 80 bar, vorzugsweise von 10 bis 60 bar. Auch Normaldruck ist möglich.

Die Hydriertemperatur liegt zweckmässig zwischen 20 und 150 ° C, vorzugsweise zwischen 40 und 80 ° C.

Die Aufarbeitung gelingt auf übliche Art durch Abtrennen vom Katalysator und Entfernen des Lösungsmittels.

Das resultierende Produkt der Formel

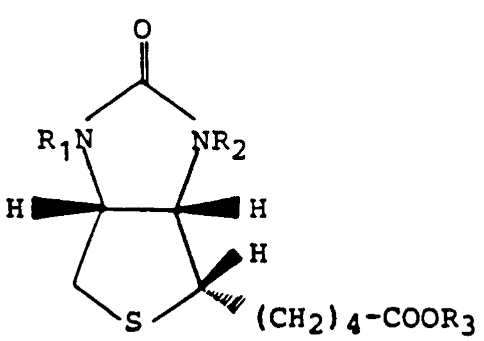

V

entspricht der optisch aktiven Vorstufe zum Biotin. Zum Freisetzen des gewünschten Produktes können die Schutzgruppen entsprechend den Lehren aus den Jap.Pat.publikationen 31669/1970 und 27279/1978 und US-PS 4 537 973 durch Behandlung mit Methansulfonsäuren unter Erwärmen abgespalten werden.

Wenn $R_3$ von Formel V eine Esterfunktion bedeutet, wird diese nach Abspaltung der Schutzgruppe unverändert vorliegen, so dass zur Gewinnung des Biotins zweckmässig eine Behandlung mit Basen wie Natriumhydroxid oder Kaliumhydroxid anschliessen sollte.

Eine weitere Methode besteht darin, V mit einer wässrigen Mineralsäure, vorzugsweise HBr, bei 30 bis 90 ° C zu behandeln. Die Abspaltung der Schutzgruppen sowie die Esterhydrolyse kann damit gleichzeitig erreicht werden.

Als Resultat der Schutzgruppenabspaltung kann, nach üblicher Aufarbeitung, das d-(+)-Biotin der Formel

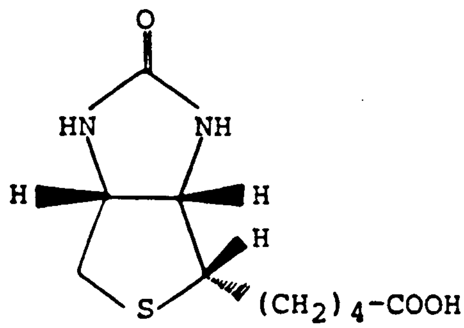

VI

erhalten werden.

## Beispiele

a 1) Herstellung von (3aS,6aR)-1-[(R)-(1-Phenylethyl)]-dihydro-1H-furo-[3,4-d]-imidazol-2,4(3H,3aH)-dion

In einen 250 ml Autoklav legte man eine Lösung von 8,98 g 1-[(R)-(1-Phenylethyl)]-1H-furo-[3,4-d]-imidazol-2,4(3H,6H)-dion (36,8 mMol) in 90 ml Dimethylformamid vor und gab 0,90 g $Rh/Al_2O_3$ (5%) zu. Dann wurde der Autoklav zweimal nacheinander mit Wasserstoff gespült, auf 40 bar aufgefüllt. Die Mischung wurde während 10 Std. gerührt. Dann wurde der Katalysator abfiltriert. Das Lösungsmittel wurde bei 13,3 mbar abgedampft und der Rückstand von 10 ml Essigsäureethylester umkristallisiert. (3aS,6aR)-1[(R)-(1-Phenylethyl)]-dihydro-1H-furo-[3,4-d]-imidazol-2,4(3H,3aH)-dion, als weisses kristallines Produkt, wurde in einer Ausbeute von 4,89 g = 54% erhalten.

Smp.: 153-154° C.

| | |
|---|---|
| $^1$H-NMR: ($CDCl_3$, 300 MHz) $\delta$ | 1,61, d, J = 7 Hz, 3H |
| | 3,45, dd, J = 10,5 Hz, 1,4 Hz, 1H |
| | 3,95, dd, J = 10,5 Hz, 5 Hz, 1H |
| | 4,21, d, J = 9,5 Hz, 1H |
| | 4,57, ddd, J = 10,5 Hz, 9,5 Hz, 1,4 Hz, 1H |
| | 5,24, bs, 1H |
| | 5,31, q, J = 7 Hz, 1H |
| | 7,4, m, 5H |
| MS (E.I. 70 ev) m/e | 246 (30%) $M^+$, 231 (45%), 161 (28%), 105 (100%). |
| IR (KBr) $cm^{-1}$ | 3388, 1771 (s), 1669 (s), 1422, 1255, 699. |
| UV (MeOH) $\lambda_{max.}$ | 372 nm, $\epsilon$ = 119 |
| | 256 nm, $\epsilon$ = 764 |

Elementaranalyse für $C_{13}H_{14}N_2O_3$ (246,27)

ber. C 63,1% H 5,7% N 11,3%

gef. C 63,4% H 5,7% N 11,4%

$[\alpha]_D^{20}$ [c = 1 $CHCl_3$] +211,7°

a 2) Herstellung von (3aS,6aR)-1-[(S)-(1-Phenylethyl)]-dihydro-1H-furo-[3,4-d]-imidazol-2,4(3H,3aH)-dion

In einen 250 ml Autoklav legte man eine Lösung von 3,7 g 1-[(S)-(1-Phenylethyl)]-1H-furo-[3,4-d]-imidazol-2,4(3H,6H)-dion (15,16 mMol) in 100 ml Essigsäure vor und gab 0,4 g Palladium auf Aktivkohle (5%) zu. Dann wurde der Autoklav zweimal nacheinander mit Wasserstoff gespült und auf 50 bar aufgefüllt. Diese Mischung wurde während 15 Std. bei Raumtemperatur gerührt. Dann wurde der Katalysator abfiltriert. Das Lösungsmittel wurde bei 20 mbar abgedampft und der Rückstand über Kieselgel mit Essigsäureethylester chromatographiert.

Eluiert wurden 2,0 g (54% Ausbeute) des Titelproduktes. Die Umkristallisation in Methanol lieferte weisse Nadeln.

Smp.: 123-125° C.

| | |
|---|---|
| $^1$H-NMR: ($CDCl_3$, 300 MHz) $\delta$ | 1,65, d, J = 7,4 Hz, 3H |
| | 4,08, d, J = 8,6 Hz, 1H |
| | 4,12, m, 1H |

4,37, dd, J = 10,3 Hz, 4,8 Hz, 1H
4,48, dd, J = 10,2 Hz, 1,3 Hz, 1H
5,36, q, J = 7,3 Hz, 1H
5,48, s, 1H
MS (E.I. 70 ev) m/e 246 (30%) $M^+$, 231 (45%), 161 (28%), 105 (100%)
$[\alpha]_D^{20}$ [c=0,5 $CHCl_3$] -6,7°
Nachher eluierte man das (3aR,6aS)-Isomer in einer Ausbeute von 1,05 g = 28%.

b 1) Herstellung von (3aS,6aR)-1-[(R)-(1-Phenylethyl)]-3-benzyldihydro-1H-furo-[3,4-d]-imidazol-2,4(3H,3aH)-dion

In einen 100 ml Dreihalskolben, ausgerüstet mit einem Magnetrührer, legte man unter Argon und unter völligem Feuchtigkeitsausschluss 48 ml Dimethoxyethan und 0,39 g Natriumhydrid (16,2 mMol) vor. Dann gab man 3,24 g (3aS,6aR)-1-[(R)-(1-Phenylethyl)]-dihydro-1H-furo-[3,4-d]-imidazol-2,4(3H,3aH)-dion (13,2 mMol) zu. Nach einer Rührzeit von 10 Min. gab man 2,76 g Benzylbromid (16,2 mMol) zu und rührte die Suspension während 30 Min. Dann wurde das Reaktionsgemisch abgedampft. Der Rückstand wurde mit 25 ml Dichlormethan und 25 ml Wasser gelöst. Die Phasen wurden getrennt und die wässrige Phase dreimal mit je 15 ml Dichlormethan gewaschen. Die organischen Phasen wurden vereinigt, mit 5 g Magnesiumsulfat getrocknet und eingedampft. (3aS,6aR)-1-[(R)-(1-Phenylethyl)]-3-benzyl-dihydro-1H-furo-[3,4-d]-imidazol-2,4-(3H,3aH)-dion konnte als beiges Produkt in einer Ausbeute von 3,56 g = 80,5% erhalten werden.
Smp.: 163-164,5°C
$^1$H-NMR: ($CDCl_3$, 300 MHz) δ 1,58, d, J = 7 Hz, 3H
3,38, dd, J = 10 Hz, 3 Hz, 1H
3,82, dd, J = 10 Hz, 5 Hz, 1H
3,89, d, J = 9 Hz, 1H
4,32, d, J = 15 Hz, 1H
4,44, ddd, J = 9 Hz, 5 Hz, 3 Hz, 1H
5,05, d, J = 15 Hz, 1H
5,36, q, J = 7 Hz, 1H
7,30-7,41, m, 10H
MS (E.I. 70 ev) m/e 336 (26%) $M^+$, 321 (9%), 231 (22%), 187 (16%), 174 (14%), 105 (56%), 91 (100%)
Elementaranalyse für $C_{20}H_{20}N_2O_3$ (336,39)
ber. C 71,4% H 6,0% N 8,3%
gef. C 71,3% H 6,2% N 8,3%
$[\alpha]_D^{20}$ [c=0,5 $CHCl_3$] +122,3°

b 2) Herstellung von (3aS,6aR)-1-[(R)-(1-Phenylethyl)]-3-(4-methoxybenzyl)-benzyl-dihydro-1H-furo-[3,4-d]-imidazol-2,4(3H,3aH)-dion

Zu einer Lösung von 50,0 g (0,2 Mol) (3aS,6aR)-1-[(R)-(1-Phenylethyl)]-dihydro-1H-furo-[3,4-d]-imidazol-2,4(3H,3aH)-dion und 39,8 g (0,25 Mol) 4-Methoxybenzylchlorid in 500 ml getrocknetem N,N-Dimethylformamid wurden in 10 Portionen während 2 Std. bei -10°C 9,75 g (0,22 Mol) Natriumhydrid (55% in Weissöl) unter Argon zugegeben. Die Reaktionsmischung wurde bei 5°C während 2 Std. und dann bei Raumtemperatur während weiteren 2 Std. gerührt. Darauf wurden 8 ml Essigsäure zugegeben. Dann wurde die Mischung zur Trockene eingedampft. Darauf wurde der Rückstand in 100 ml Wasser und 200 ml Dichlormethan aufgenommen, die Phasen getrennt und die wässrige Phase 2 mal mit 100 ml Dichlormethan extrahiert. Die organischen Phasen wurden über Magnesiumsulfat getrocknet und eingeengt.
Nach Aufschlämmen in Ethanol unter Rückfluss, Abkühlen und Filtrieren erhielt man 53,5 g = 72% des Titelproduktes in Form von weissen Nadeln.
Smp.: 146,1-146,4°C.
$^1$H-NMR: ($CDCl_3$, 300 Hz) δ 1,58, d, J = 7 Hz, 3H
3,37, dd, J = 10 Hz, 3 Hz, 3H
3,82, s, 3H
3,82, dd, J = 10 Hz, 5,5 Hz, 1H
3,88, d, J = 8,5 Hz, 1H
4,25, d, J = 14,5 Hz, 1H
4,34, ddd, J = 8,5 Hz, 5,5 Hz, 3H

4,97, d, J = 14,5 Hz, 1H
5,34, q, J = 7 Hz, 1H
6,88, d, J = 8,5 Hz, 2H
7,32-7,38, m, 7H

$[\alpha]_D^{20}$ [c = 1 $CHCl_3$] + 104,7°

b 3) Herstellung von (3aS,6aR)-[(R)-(1-Phenylethyl)]-3-tert.butoxycarbonyl-dihydro-1H-furo-[3,4-d]-imidazol-2,4(3H,3aH)-dion

Zu einer Lösung von 20,0 g (81 mMol) (3aS,6aR)-[(R)-(1-Phenylethyl)]-dihydro-1H-furo-[3,4-d]-imidazol-2,4(3H,3aH)-dion und 21,3g (97 mMol) Di-tert.-butyldicarbonat in 200 ml getrocknetem N,N-Dimethylformamid wurden in 10 Portionen während 2 Std. bei -10° C 3,83g (88 mMol) Natriumhydrid (55% in Weissöl) unter Argon zugegeben. Die Reaktionsmischung wurde bei 5° C während 2 Std. und dann bei Raumtemperatur während weiteren 2 Std. gerührt. Darauf wurden 1 ml Essigsäure zugegeben. Dann wurde die Mischung zur Trockene eingedampft. Darauf wurde der Rückstand in 50 ml Wasser und 100 ml Dichlormethan aufgenommen, die Phasen getrennt und die wässrige Phase 2 mal mit 100 ml Dichlormethan extrahiert. Die organischen Phasen wurden über Magnesiumsulfat getrocknet und eingeengt.
Nach Aufschlämmen in Ethanol unter Rückfluss, Abkühlen und Filtrieren erhielt man 25,8 g = 92% des Titelproduktes in Form von weissen Nadeln.
Smp.: 177,4-178,1° C.

$^1$H-NMR: ($CDCl_3$, 300 MHz) δ
1,59, s, 9H
1,63, d, J = 7,5 Hz, 3H
3,51, d, J = 11 Hz, 1H
3,97, dd, J = 11 Hz, 5 Hz, 1H
4,50, dd, J = 8 Hz, 5 Hz, 1H
4,90, d, J = 8 Hz, 1H
5,39, q, J = 7,5 Hz, 1H
7,3-7,4, m, 5H

$[\alpha]_D^{20}$ [c = 1 $CHCl_3$] + 55,8°

b 4) Herstellung von (3aS,6aR)-[(R)-(1-Phenylethyl)]-3-methoxymethyl-dihydro-1H-furo-[3,4-d]-imidazol-2,4-(3H,3aH)-dion

Zu einer Lösung von 19 g (77 mMol) (3aS,6aR)-[(R)-(1-Phenylethyl)]-dihydro-1H-furo-[3,4-d]-imidazol-2,4(3H,3aH)-dion und 9,42 g (120 mMol) Chlormethylmethylether in 200 ml getrocknetem N,N-Dimethylformamid wurden in 10 Portionen während 2 Std. bei -10° C 4,0 g (93 mMol) Natriumhydrid (55% in Weissöl) unter Argon zugegeben. Die Reaktionsmischung wurde bei 5° C während 2 Std. und dann bei Raumtemperatur während weiteren 2 Std. gerührt. Darauf wurden 2 ml Essigsäure zugegeben. Dann wurde die Mischung zur Trockene eingedampft. Darauf wurde der Rückstand in 50 ml Wasser und 100 ml Dichlormethan aufgenommen, die Phasen getrennt und die wässrige Phase 2 mal mit 100 ml Dichlormethan extrahiert. Die organischen Phasen wurden über Magnesiumsulfat getrocknet und eingeengt.
Nach Chromatographieren des öligen Rückstandes über Kieselgel mit 500 ml Dichlormethan/Essigsäuremethylester und Einengen der Fraktionen wurden 4,0 g = 18% des Titelproduktes als weisses Pulver erhalten.
Smp.: 96-98° C.

$^1$H-NMR ($CDCl_3$, 300 MHz) δ
1,61, d, J = 7,5 Hz, 3H
3,36, s, 3H
3,41, dd, J = 10 Hz, 3 Hz, 1H
3,89, dd, J = 10 Hz, 6 Hz, 1H
4,36, d, J = 9 Hz, 1H
4,52, ddd, J = 9 Hz, 6 Hz, 3 Hz, 1H
4,87, d, J = 11 Hz, 1H
4,97, d, J = 11 Hz, 1H
5,34, q, J = 7,5 Hz, 1H
7,35-7,4, m, 5H

c) Herstellung von (3aS,6aR)-1-[(R)-(1-Phenylethyl)]-3-benzyldihydro-1H-thieno-[3,4-d]-imidazol-2,4(3H,3aH)-dion

In einen 25 ml Kolben, der mit einem Magnetrührer und einem Kugelkühler ausgerüstet war, legte man 2,03 g (3aS,6aR)-1-[(R)-(1-Phenylethyl)]-3-benzyl-dihydro-1H-furo-[3,4-d]-imidazol-2,4(3H,3aH)-dion (6,03 mMol) gelöst in 2 ml Dimethylacetamid vor. Man heizte die Lösung auf 150°C und gab 0,81 g Kaliumthioacetat (7,14 mMol) zu. Nach 45 Min. liess man das Reaktionsgemisch erkalten und behandelte mit 40 ml Toluol und 40 ml Wasser. Die Phasen wurden getrennt, die Toluol-Phase wusch man dreimal mit 20 ml Wasser und die vereinigten wässrigen Phasen dreimal mit je 30 ml Toluol. Die Toluol-Phasen wurden vereinigt, getrocknet und eingedampft. Der so erhaltene braune Feststoff wurde mit 5 ml Ether gewaschen. Dann filtrierte man das beige Produkt (3aS,6aR)-1-[(R)-(1-Phenylethyl)]-3-benzyl-dihydro-1H-thieno-[3,4-d]-imidazol-2,4(3H,3aH)-dion ab und trocknete es.
Ausbeute: 1,82 g = 85%.
Smp.: 144-145°C

| | |
|---|---|
| $^1$H-NMR: ($CDCl_3$, 300 MHz) $\delta$ | 1,67, d, J = 7 Hz, 3H |
| | 2,71, dd, J = 12,5 Hz, 2,5 Hz, 1H |
| | 3,03, dd, J = 12,5 Hz, 5 Hz, 1H |
| | 3,81, d, J = 8 Hz, 1H |
| | 4,34, d, J = 15 Hz, 1H |
| | 4,40, ddd, J = 8 Hz,5 Hz,2,5 Hz,1H |
| | 5,04, d, J = 15 Hz, 1H |
| | 5,41, q, J = 7 Hz, 1H |
| | 7,30-7,50, m, 10H |
| MS (E.I. 70 ev) m/e | 352 (1%) $M^+$, 324 (30%), 278 (35%), 174 (80%), 146 (30%), 105 (70%), 91 (100%) |

Elementaranalyse von $C_{20}H_{20}N_2O_2S$ (352,46)
ber. C 68,2% H 5,7% N 7,9% S 9,1%
gef. C 67,9% H 5,9% N 8,0%
$[\alpha]_D^{20}$ [c=0,5 $CHCl_3$] +128,5°

d 1) Herstellung von (3aS,6aR)-Hexahydro-1-[(R)-(1-phenylethyl)]-2-oxo-3-benzylthieno-[3,4-d]-imidazol-4-ylidenpentansäure

In einen 25 ml Rundkolben legte man 159,8 mg Natriumhydrid (3,66 mMol) und 1,7 ml Dimethylsulfoxid vor. Man erwärmte die Suspension unter Rühren und unter Argon auf 70°C. Es wurde während 40 Min. weitergerührt, bis keine Wasserstoffentwicklung mehr sichtbar war. Man kühlte die Lösung auf Raumtemperatur ab und gab eine Lösung von 801,5 mg (4-Carboxybutyl)-triphenylphosphoniumbromid (1,8 mMol) in 1 ml Dimethylsulfoxid zu. Das dunkelrote Reaktionsgemisch wurde während 15 Min. gerührt und dann zu einer Lösung aus 271 mg (3aS,6aR)-1-[(R) (1-phenylethyl)]-3-benzyl-dihydro-1H-thieno-]3,4-d]-imidazol-2,4-(3H,-3aH)-dion (0,77 mMol) in 2 ml Dimethylsulfoxid und 0,2 ml Toluol zugetropft. Das Reaktionsgemisch wurde während 2 Std. bei Raumtemperatur gerührt. Dann gab man 1 g Eis, 1 ml konz. HCl und nochmals 9 g Eis dazu. Nach 5 Min. wurden 5 ml Wasser, 10 ml Benzol und 5 ml Essigsäureethylester dazugegeben. Nun wurde die Mischung während l Std. bei 60°C gerührt. Die Phasen wurden getrennt. Die braune organische Phase wurde mit 5 g Magnesiumsulfat getrocknet und mit 4 präparativen Kieselgel-Dünnschicht-Platten(1 mm) mittels Essigsäureethylester getrennt.
Das Produkt (3aS,6aR)-Hexahydro-1-[(R)-(1-phenylethyl)]-2-oxo-3-benzylthieno-[3,4-d]-imidazol-4-ylidenpentansäure, ein farbloses Oel, konnte in einer Ausbeute von 38,2 mg = 12% erhalten werden.

| | |
|---|---|
| $^1$H-NMR: ($CDCl_3$, 300 MHz) $\delta$ | 1,58, d, J = 7 Hz, 3H |
| | 1,59, q, J = 7 Hz, 2H |
| | 1,98, m, 2H |
| | 2,22, t, J = 7,5 Hz, 2H |
| | 2,29, dd, J = 11,5 Hz, 4 Hz, 1H |
| | 2,41, dd, J = 11,5 Hz, 5 Hz, 1H |
| | 3,97, d, J = 15 Hz, 1H |
| | 4,18, m, 2H |
| | 4,84, d, J = 15 Hz, 1H |
| | 5,30, q, J = 7 Hz, 1H |
| | 5,31, t, J = 7 Hz, 1H |
| | 7,10-7,40, m, 10H |
| MS (E.I. 70 ev) m/e | 436 (55%) $M^+$, 331 (55%), 252 (32%), 237 (60%), 120 (40%), 106 (100%). |

d 2) Herstellung von (3aS,6aR)-Hexahydro-1-[(R)-(1-phenylethyl)]-2-oxo-3-benzylthieno-[3,4-d]-imidazol-4-ylidenpentansäure

0,802 g (33 mMol) Magnesiumspäne wurden in 5 ml Tetrahydrofuran vorgelegt. Dann gab man 2,37 g (11 mMol) Dibrombutan in 30 ml Tetrahydrofuran innerhalb 1 Std. zu. Das Reaktionsgemisch wurde über 2 Std. am Rückfluss gehalten, dann gab man 2,55 g (22 mMol) Tetramethylethylendiamin zu und hielt eine weitere Stunde am Rückfluss. Der auf 0 °C gekühlten Suspension wurden darauf 3,52 g (10 mMol) (3aS,6aR)-1-[(R)-(1-phenylethyl)]-3-benzyl-dihydro-1H-thieno-[3,4-d]-imidazol-2,4(3H,3aH)-on in 50 ml Tetrahydrofuran während 20 min zudosiert. Danach wurde das Reaktionsgemisch während 2 Std. bei Raumtemperatur gerührt und darauf auf 0 °C abgekühlt. Während 1 Std. bei 0 °C und 1 Std. bei Raumtemperatur leitete man Kohlendioxidgas ein.
Das Reaktionsgemisch wurde auf ein Gemisch von 85 g Eis und 11,5 ml konz. Salzsäure gegossen und darauf mit Essigsäureethylester extrahiert. Die vereinigten organischen Phasen wurden mit Wasser und gesättigter Kochsalzlösung gewaschen, mit Magnesiumsulfat getrocknet und schliesslich eingeengt.
Dem Rückstand, aufgenommen in 170 ml Toluol, wurden 50 mg p-Toluolsulfonsäure zugegeben. Man erhitzte zum Rückfluss und destillierte mittels eines Wasserabscheiders das Reaktionswasser ab. Die verbleibende Toluol-Lösung wurde eingeengt und das erhaltene Oel über Kieselgel mit Essigsäureethylester/Toluol chromatographiert.
Man erhielt 1,22 g = 28% des Titelproduktes als leicht gelbliches Oel.

e) Herstellung von (3aS,6aR)-Hexahydro-1-[(R)-(1-phenylethyl)] 2-oxo-3-benzylthieno-[3,4-d]-imidazol-4-yl-pentansäure

In einen 100 ml Autoklav legte man eine Lösung von 78,6 mg (3aS,6aR)-Hexahydro-1-[(R)-(1-phenylethyl)]-2-oxo-3-benzylthieno-[3,4-d]-imidazol-4-ylidenpentansäure in 5 ml Isopropanol vor und gab 39 mg Palladium auf Kohle 5% zu. Man spülte den Autoklav zweimal mit Wasserstoff und rührte die Mischung unter 50 bar Wasserstoffdruck bei 50 °C während 24 Std. Dann filtrierte man den Katalysator ab und dampfte das Lösungsmittel ab. Man erhielt das Produkt (3aS,6aR)-Hexahydro-1-[(R)-(1-phenylethyl)]-2-oxo-3-benzylthieno-[3,4-d]-imidazol-4-yl-pentansäure als farbloses Oel in einer Ausbeute von 56,1 mg = 72%.

| | |
|---|---|
| $^1H$-NMR: ($CDCl_3$, 300 MHz) $\delta$ | 1,57, m, 6H |
| | 1,61, d, J = 7 Hz, 3H |
| | 2,13, m, 1H |
| | 2,33, m, 2H |
| | 3,03, m, 1H |
| | 3,90, dd, J = 10 Hz, 5 Hz, 1H |
| | 3,94, d, J = 15 Hz, 1H |
| | 4,22, m, 1H |
| | 5,06, d, J = 15 Hz, 1H |
| | 5,28, q, J = 7 Hz, 1H |
| | 7,20-7,40, m, 10H |
| MS (E.I. 70 ev) m/e | 438 (13%), 423 (6%), 333 (16%), 187 (30%) 174 (15%), 105 (63%), 91 (100%). |

f) Herstellung von d-Biotin

In einem 25 ml Rundkolben erhitzte man eine Lösung von 100 mg (3aS,6aR)-Hexahydro-1-[(R)-(1-phenylethyl)]-2-oxo-3-benzylthieno-[3,4-d]-imidazol-4-yl-pentansäure in 4 ml Bromwasserstoffsäure (48%ig) während 3 Std. bei 120 °C, mit einem Vakuum von 400 mbar. Nachdem das Reaktionsgemisch abgekühlt wurde, extrahierte man es mit 5 ml Toluol. Darauf wurde die wässrige Phase im Vakuum abgedampft. Der Rückstand wurde in 10 ml Wasser gelöst und mit 10 ml Chloroform bei 60 °C extrahiert. Die wässrige Phase wurde bis 1 ml eingeengt und abgekühlt. d-(+)-Biotin fiel in 40 mg beigen Kristallen aus = 72% Ausbeute.
Smp.: 227-229 °C
$[\alpha]_D^{25}$ [c = 0,1 1N NaOH] +84,5 °.

**Patentansprüche**

**1.** Verfahren zur Herstellung von (+)-Biotin, dadurch gekennzeichnet, daß man Verbindungen der Formel

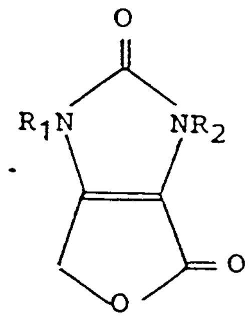

I

worin $R_1$ eine (R)- oder (S)-1-Phenylethylgruppe und $R_2$ Wasserstoff, eine Acetyl- oder Propionylgruppe, eine Benzoylgruppe, eine Benzylgruppe oder p-Methoxybenzylgruppe, eine Ethoxycarbonylgruppe, eine tert.-Butoxycarbonylgruppe, eine Benzyloxycarbonylgruppe, eine Phenoxycarbonylgruppe, eine Methoxymethylgruppe, eine Pyranylgruppe, eine Benzolsulfonylgruppe, eine p-Toluolsulfonylgruppe, eine Methylsulfonylgruppe, eine Diphenylphosphinylgruppe, eine Diethoxyphosphinylgruppe oder eine Trimethyl- oder tert.-Butyl-dimethylsilylgruppe bedeuten, katalytisch mit Wasserstoff hydriert, das gewünschte Diastereomer der Formel

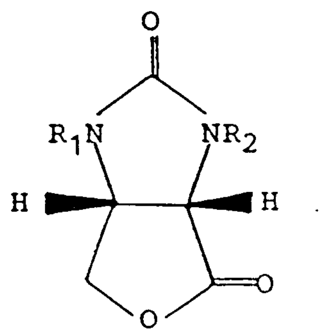

II

abtrennt, wenn $R_2$ = H die Schutzgruppe durch Umsetzung mit entsprechenden aliphatischen oder aromatischen Säurechloriden, aliphatischen oder aromatischen Carbonsäureanhydriden, Halogenameisensäurealkylestern, Benzylhalogeniden, 1-Alkoxyalkylhalogeniden, Enolethern, aromatischen oder aliphatischen Sulfonsäurehalogeniden, Diarylphosphinsäurehalogeniden, Phosphorsäuredialkylesterchloriden, Trialkylsilylhalogeniden oder Trialkylsilylacetamiden einführt, und weiter auf bekannte Weise durch Umsetzung mit einem Thiocarbonsäuresalzderivat in das entsprechende Thiolacton überführt, dieses entweder mit einer Grignard-Reaktion und anschließender Wasserabspaltung oder mit einer Verbindung der Formel

$(C_6H_5)_3P^{\oplus}(CH_2)_4COOR_3.X^{\ominus}$ III

worin $R_3$ = H oder Alkyl mit 1 bis 4 C-Atomen und X ein Halogenatom bedeuten, in Gegenwart einer Base zu einer Verbindung der Formel

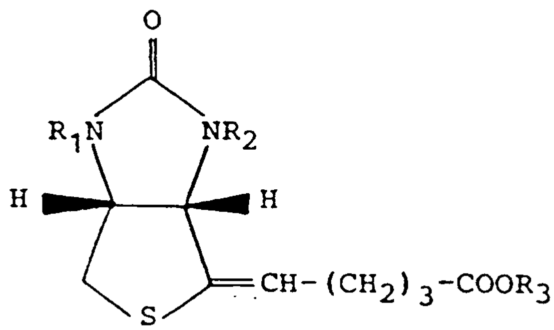

IV

worin $R_3$ oben genannte Bedeutung hat, umsetzt, in einem nächsten Schritt diese Verbindung katalytisch mit Wasserstoff hydriert und schließlich durch Abspalten der Schutzgruppen in das Endprodukt überführt.

**2.** Verfahren nach Anspruch 1, dadurch gekennzeichnet, dass man als Hydrierkatalysatoren für die Hydrierung von I zu II Platin-, Palladium-, Rhodium- oder Rutheniumkatalysatoren auf Trägermaterialien, wie Kohle, Aluminiumoxid, Aluminiumsilikat, Siliciumdioxid, Calciumcarbonat oder Homogenkatalysatoren, bestehend aus Rhodium oder Iridium mit Triphenylphosphin oder Cyclooctadien als Liganden, anwendet.

**3.** Verfahren nach Ansprüchen 1 und 2, dadurch gekennzeichnet, dass man als Hydrierkatalysator für die Hydrierung von I zu II Rhodium auf Aluminiumoxid anwendet.

**4.** Verfahren nach Ansprüchen 1 bis 3, dadurch gekennzeichnet, dass die Verbindung II in der Position 3aS- und in der Position 6aR-Konfiguration aufweist.

**5.** Verfahren nach Ansprüchen 1 bis 4, dadurch gekennzeichnet, dass man als Thiocarbonsäuresalzderivat Erdalkali- oder Alkalithiocarbonsäuresalze von aliphatischen oder aromatischen Carbonsäuren verwendet.

**6.** Verfahren nach Ansprüchen 1 bis 5, dadurch gekennzeichnet, dass man als Thiocarbonsäuresalzderivat Alkalithioacetate einsetzt.

**7.** Verfahren nach Ansprüchen 1 bis 6, dadurch gekennzeichnet, dass die Grignard Reaktion mit einer Grignard-Verbindung der Formel

$XMg-(CH_2)_4-MgX$

worin X = Chlor oder Brom bedeutet, anschliessender Behandlung mit $CO_2$ und darauffolgender Wasserabspaltung in Gegenwart einer Säure erfolgt.

**8.** Verfahren nach Ansprüchen 1 bis 7, dadurch gekennzeichnet, dass III in Gegenwart von Alkylalkalimetallen, Alkalimetallhydriden, Verbindungen der Formel

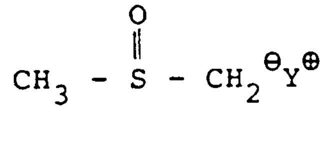

worin Y ein Alkalimetallatom bedeutet, Alkalimetallalkoxiden, Alkalimetallalkylamiden, Alkalimetallamiden, Alkalihydroxiden oder auch Carbonaten, zu IV umgesetzt wird.

**9.** Verfahren nach Ansprüchen 1 bis 8, dadurch gekennzeichnet, dass man die Hydrierung von IV in Gegenwart eines Palladium-, Platin-, Rhodium-, Ruthenium- oder Nickelkatalysators, gegebenenfalls auf

Trägermaterialien wie Kohle, Aluminiumoxid, Aluminiumsilikat, Siliciumdioxid, Calciumcarbonat, durchführt.

**10.** Verfahren nach Ansprüchen 1 bis 9, dadurch gekennzeichnet, dass die Abspaltung der Schutzgruppen durch Behandlung mit Säuren erfolgt.

**Claims**

**1.** Process for the preparation of ( + )-biotin, characterized in that compounds of the formula

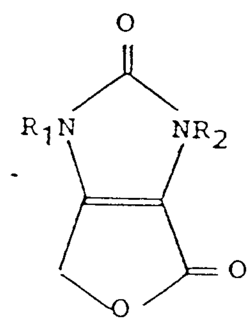

I

wherein $R_1$ is an (R)- or (S)-1-phenylethyl group and $R_2$ is hydrogen, an acetyl or propionyl group, a benzoyl group, a benzyl group or p-methoxybenzyl group, an ethoxycarbonyl group, a t-butoxycarbonyl group, a benzyloxycarbonyl group, a phenoxycarbonyl group, a methoxymethyl group, a pyranyl group, a benzenesulfonyl group, a p-toluenesulfonyl group, a methylsulfonyl group, a diphenylphosphinyl group, a diethoxyphosphinyl group or a trimethyl or t-butyldimethylsilyl group, are catalytically hydrogenated with hydrogen, the desired diastereomer of the formula

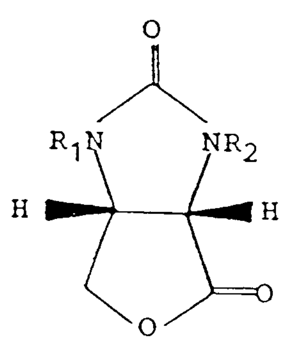

II

is separated, when $R_2 = H$, the protective group is introduced by reaction with the corresponding aliphatic or aromatic acid chlorides, aliphatic or aromatic carboxylic acid anhydrides, halogenated formic acid alkyl esters, benzylhalides, 1-alkoxyalkyl halides, enolethers, aromatic or aliphatic sulfonic acid halides, diarylphosphinic acid halides, phosphoric acid dialkyl ester chlorides, trialkylsilyl halides or trialkylsilyl acetamides, and the conversion into the corresponding thiolactone is then effected in per se known manner by reaction with a thiocarboxylic acid salt derivative, said thiolactone is reacted either by a Grignard-reaction and with subsequent dehydration or with a compound of the formula

$(C_6H_5)_3P^{\oplus}(CH_2)_4COOR_3 . X^{\ominus}$ III

wherein $R_3$ is H or alkyl with 1 to 4 carbon atoms and X is a halogen atom, in the presence of a base to obtain a compound of the formula

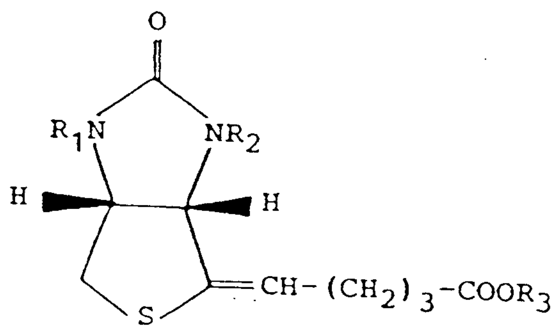

IV

wherein $R_3$ is defined as above, in the next step said compound is catalytically hydrogenated and finally it is converted into the end product by removal of the protective groups.

**2.** Process according to claim 1, characterized in that as hydrogenation catalysts for the hydrogenation of I to II platinum, palladium, rhodium or ruthenium catalysts on carrier materials, such as carbon, aluminum oxide, aluminum silicate, silicon dioxide, calcium carbonate, or homogeneous catalysts, consisting of rhodium or iridium with triphenyl phosphine or cyclooctadiene as ligands, are used.

**3.** Process according to claims 1 and 2, characterized in that as hydrogenation catalyst for the hydrogenation of I to II rhodium on aluminum oxide is used.

**4.** Process according to claims 1 to 3, characterized in that compound II has the 3aS,6aR-configuration.

**5.** Process according to claims 1 to 4, characterized in that as thiocarboxylic acid salt derivative alkaline earth or alkali thiocarboxylic acid salts of aliphatic or aromatic carboxylic acids are used.

**6.** Process according to claims 1 to 5, characterized in that as thiocarboxylic acid salt derivative alkali thioacetate is used.

**7.** Process according to claims 1 to 6, characterized in that the Grignard-reaction is carried out with a Grignard-compound of the formula

$XMg-(CH_2)_4-MgX$

wherein X is chlorine or bromine, with subsequent treatment with $CO_2$ and dehydration in the presence of an acid.

**8.** Process according to claims 1 to 7, characterized in that III is reacted to IV in the presence of alkyl alkali metals, alkali metal hydrides, compounds of the formula

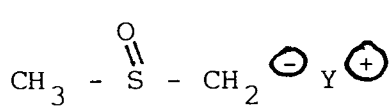

wherein Y is an alkali metal atom, alkali metal alkoxides, alkali metal alkylamides, alkali metal amides, alkali hydroxides or also carbonates.

**9.** Process according to claims 1 to 8, characterized in that the hydrogenation of IV is carried out in the presence of a palladium, platinum, rhodium, ruthenium or nickel catalyst, if desired on carrier materials, such as carbon, aluminum oxide, aluminum silicate, silicon dioxide, calcium carbonate.

**10.** Process according to claims 1 to 9, characterized in that the removal of the protective groups is carried out by treatment with acids.

## Revendications

1. Procédé de préparation de la (+)-biotine, caractérisé en ce que l'on hydrogène catalytiquement avec l'hydrogène des composés de formule

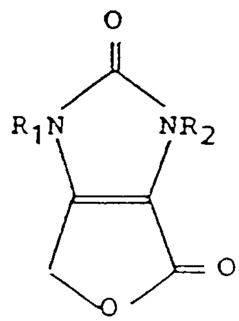

I

dans laquelle $R_1$ représente un groupe (R)- ou (S)-1-phényléthyle et $R_2$ représente un atome d'hydrogène, un groupe acétyle ou propionyle, un groupe benzoyle, un groupe benzyle ou un groupe p-méthoxybenzyle, un groupe éthoxycarbonyle, un groupe tert.butoxycarbonyle, un groupe benzyloxycarbonyle, un groupe phénoxycarbonyle, un groupe méthoxyméthyle, un groupe pyranyle, un groupe benzènesulfonyle, un groupe p-toluènesulfonyle, un groupe méthylsulfonyle, un groupe diphénylphosphinyle, un groupe diéthoxyphosphinyle ou un groupe triméthyl- ou tert.butyldiméthylsilyle, on sépare le diastéréoisomère souhaité de formule

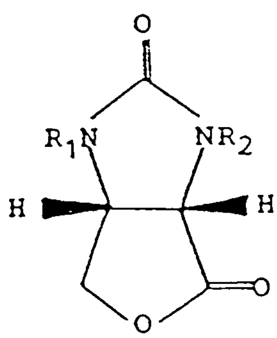

II

lorsque $R_2$ = H on introduit le groupe protecteur par réaction avec des chlorures d'acides aliphatiques ou aromatiques, des anhydrides d'acides carboxylique aliphatiques ou aromatiques, des halogénoformiates d'alkyle, des halogénures de benzyle, des halogénures de 1-alcoxyalkyle, des énoléthers, des halogénures d'acides sulfoniques aromatiques ou aliphatiques, des halogénures d'acides diarylphosphiniques, des chlorures de phosphates de dialkyle, des halogénures de trialkylsilyle ou des trialkylsilylacétamides correspondants, puis on les convertit de manière connue par réaction avec un dérivé de sel d'acide thiocarboxylique en la thiolactone correspondante, que l'on convertit soit par une réaction de Grignard puis élimination d'eau soit par réaction avec un composé de formule

$(C_6H_5)_3P^{\oplus}(CH_2)_4COOR_3.X^{\ominus}$    III

dans laquelle $R_3$ = H ou alkyle de 1 à 4 atomes de carbone et X représente un atome d'halogène, en présence d'une base, en un composé de formule

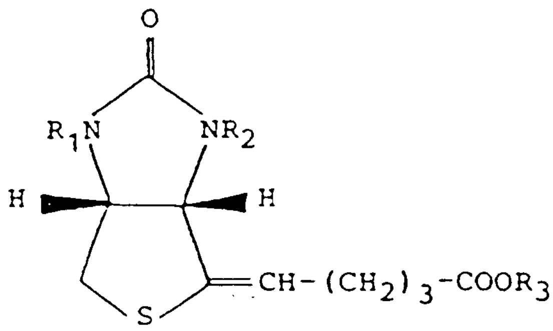

IV

dans laquelle $R_3$ a la signification indiquée ci-dessus, dans une étape suivante on hydrogène catalytiquement ce composé avec de l'hydrogène et enfin on le convertit en le produit final par élimination des groupes protecteurs.

**2.** Procédé selon la revendication 1, caractérisé en ce que l'on utilise comme catalyseur d'hydrogénation pour l'hydrogénation de I en II des catalyseurs au platine, au palladium, au rhodium ou au ruthénium sur des matériaux de support comme le charbon, l'oxyde d'aluminium, le silicate d'aluminium, le dioxyde de silicium, le carbonate de calcium ou des catalyseurs homogènes consistant en rhodium ou en iridium avec comme ligands la triphénylphosphine ou le cyclooctadiène.

**3.** Procédé selon les revendications 1 et 2, caractérisé en ce que l'on utilise comme catalyseur d'hydrogénation pour l'hydrogénation de I en II le rhodium sur oxyde d'aluminium.

**4.** Procédé selon les revendications 1 à 3, caractérisé en ce que le composé II présente la configuration S en position 3a et la configuration R en position 6a.

**5.** Procédé selon les revendications 1 à 4, caractérisé en ce que l'on utilise comme dérivé de sel d'acide thiocarboxylique des thiocarboxylates alcalinoterreux ou alcalins d'acides carboxyliques aliphatiques ou aromatiques.

**6.** Procédé selon les revendications 1 à 5, caractérisé en ce que l'on utilise comme dérivé de sel d'acide thiocarboxylique des thioacétates alcalins.

**7.** Procédé selon les revendications 1 à 6, caractérisé en ce que la réaction de Grignard a lieu avec un composé de Grignard de formule

$XMg-(CH_2)_4-MgX$

dans laquelle x représente un atome de chlore ou de brome, puis avec un traitement avec $CO_2$ et ensuite élimination de l'eau en présence d'un acide.

**8.** Procédé selon les revendications 1 à 7, caractérisé en ce que III est converti en IV en présence d'alkyl-métaux alcalins, d'hydrures de métaux alcalins, de composés de formule

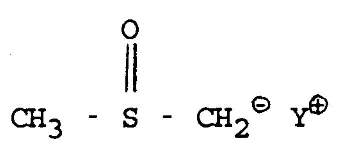

dans laquelle Y représente un atome de métal alcalin, d'oxydes de métaux alcalins, d'alkylamidures de métaux alcalins, d'amidures de métaux alcalins, d'hydroxydes alcalins ou aussi de carbonates.

**9.** Procédé selon les revendications 1 à 8, caractérisé en ce que l'on réalise l'hydrogénation de IV en

présence d'un catalyseur au palladium, au platine, au rhodium, au ruthénium ou au nickel, éventuellement sur des matériaux de support comme le charbon, l'oxyde d'aluminium, le silicate d'aluminium, le dioxyde de silicium, le carbonate de calcium.

**10.** Procédé selon les revendications 1 à 9, caractérisé en ce que l'élimination des groupes protecteurs a lieu par traitement avec des acides.